# EUROPÄISCHE PATENTANMELDUNG

(11) **EP 3 640 377 A1**
(43) Veröffentlichungstag der Anmeldung: **22.04.2020**
(21) Anmeldenummer: 19208767.4
(22) Anmeldetag: 13.03.2015
(51) Int. Cl.: D01F 1/10, C08K 5/00

(54) **FASERN UND GARNE MIT SCHMERZ REDUZIERENDEN WIRKSTOFF**

(30) Priorität: 19.03.2014 DE 102014004258
(62) Teilanmeldung aus: 15710478.7
(71) Anmelder: Bauerfeind AG, 07937 Zeulenroda-Triebes (DE)
(72) Erfinder: Bauerfeind, Hans B., 07937 Zeulenroda-Triebes (DE)
(74) Vertreter: Wohlfahrt, Jan Günther

(57) **Zusammenfassung**

Garn enthaltend einen Formkörper umfassend eine Polymerzusammensetzung, enthaltend ein Polymer und einem Schmerz oder Juckreiz reduzierenden Wirkstoff.

## Beschreibung

Die vorliegende Erfindung betrifft eine Polymerzusammensetzung, enthaltend ein Polymer und mindestens einen Wirkstoff, Formkörper mit einer solchen Polymerzusammensetzung, die Verwendung der Formkörper und Polymerzusammensetzungen und entsprechende Garne, Vliesstoffe, Kleidungsstücke und medizinische Hilfsmittel.

Polymerzusammensetzungen, die durch Additive "funktionalisiert" sind, sind bekannt. So beschreibt die US-A 5,766,746 beispielsweise Cellulosefasern, die eine flammwidrige Komponente enthalten. Die DE 100 37 983 A1 beschreibt Polymerzusammensetzungen mit einem Gehalt an Alkaloid und die DE 100 07 794 A1 Polymerzusammensetzungen mit einem Material aus Meerespflanzen oder -tieren.

Solche Polymerzusammensetzungen werden zu Garnen, textilen Flächen und Kleidungsstücken weiterverarbeitet.

Der Zusatz von Additiven zur Funktionalisierung von Polymerfasern führt oftmals zu Problemen, die bei der Verwendung der Fasern notwendigen Eigenschaften, insbesondere mechanische Festigkeit, Schlingenfestigkeit, Faserdehnung, Scheuereigenschaft und Anfärbbarkeit, dennoch zu erhalten.

Es besteht aber eine stetige Nachfrage nach funktionellen Fasern und Kleidungsstücken aus solchen Fasern mit spezifischen Additiven, so dass es wichtig ist, solche Fasern bereitzustellen, bei denen die beschriebenen Schwierigkeiten überwunden werden, damit funktionalisierte Fasern die entsprechenden Anforderungen an das aus ihnen produzierte Kleidungsstück erfüllen können.

Für den Markt besonders relevante Additive sind dabei Additive mit einer Wirkung auf den Organismus des Trägers der aus den Polymerzusammensetzungen hergestellten Kleidungsstücke.

Bisher bekannte Strukturen beinhalten nur geringe Konzentrationen dieser Stoffe. Diese Stoffe werden durch Tränkung oder Beschichtung auf die Trägermatrix aufgetragen. Dieses führt beispielsweise zu geringen Waschbeständigkeiten, sodass solche Stoffe regelmäßig zur Aufrechterhaltung ihrer Wirkweise neu zugeführt werden müssen.

Daher liegt der vorliegenden Erfindung das technische Problem zugrunde, eine Polymerzusammensetzung bereitzustellen, die ein entsprechendes Additiv enthält, wobei dennoch eine gute Stabilität und Verarbeitbarkeit der aus der Polymerzusammensetzung hergestellten Formkörper resultiert.

Bei eng an der Haut anliegenden Kleidungsstücken und medizinischen Hilfsmitteln, beispielsweise Bandagen und Kompressionsstrümpfen, spielt beim Tragekomfort eine Regulierung der Hautfeuchtigkeit eine Rolle, insbesondere sollte ein Austrocknen der Haut unterbunden werden. Auch sollen solche textile Flächen nicht zu Hautaustrocknung und zu Hautreizungen führen oder bereits bestehende Hauterkrankungen wie Neurodermitis verstärken. Dabei soll auch ein mit solchen Hautkrankheiten verbundener Juckreiz oder Schmerz zumindest verringert werden.

Das der vorliegenden Erfindung zugrunde liegende technische Problem ist auch die Bereitstellung von insbesondere in dieser Hinsicht verbesserten Kleidungsstücken und medizinischen Hilfsmitteln und deren Ausgangsmaterial.

Die vorliegende Erfindung löst das ihr zugrunde liegende technische Problem durch den Gegenstand der unabhängigen Ansprüche.

Insbesondere löst die vorliegende Erfindung das ihr zugrunde liegende technische Problem durch eine Polymerzusammensetzung, enthaltend ein Polymer und mindestens einen Wirkstoff ausgewählt aus der Gruppe bestehend aus einem eine Okklusion erzeugenden Wirkstoff, insbesondere einem eine innere Okklusion erzeugenden Wirkstoff oder einem eine äußere Okklusion erzeugenden Wirkstoff, einem feuchtigkeitsspendenden Wirkstoff, einem Schmerz oder Juckreiz reduzierenden Wirkstoff und Mischungen davon.

Die Polymerzusammensetzung enthält also erfindungsgemäß neben dem Polymer zusätzlich mindestens einen Wirkstoff, wobei es sich bei dem Wirkstoff um einen eine Okklusion erzeugenden Wirkstoff, insbesondere einen eine innere Okklusion erzeugenden Wirkstoff oder einen eine äußere Okklusion erzeugenden Wirkstoff, um einen feuchtigkeitsspendenden Wirkstoff, oder um einen Schmerz oder Juckreiz reduzierenden Wirkstoff handeln kann.

Natürlich sind auch das Vorhandensein von zwei oder mehreren dieser Wirkstoffe und Mischungen dieser Wirkstoffe von der Erfindung erfasst.

Es zeigte sich überraschender Weise, dass solche Wirkstoffe in eine Polymerzusammensetzung eingebracht werden können und dort als Additiv ihre Wirkung, insbesondere direkt auf der anliegenden Haut entfalten können, wenn die Polymerzusammensetzung in Form von entsprechenden Produkten wie Kleidungsstücken und medizinischen Hilfsmitteln an der Haut anliegt. Insbesondere können diese Wirkstoffe dabei in vorteilhafter Weise ein Austrocknen der Haut verhindern und/oder Schmerz und Juckreiz bei Krankheiten wie Neurodermitis zumindest vermindern. Auch können die Wirkstoffe dazu verwendet werden, weitere Wirkstoffe in die Haut eindringen zu lassen.

Dabei konnte überraschender Weise auch festgestellt werden, dass die in der Polymerzusammensetzung als Additive enthaltene erfindungsgemäße Gruppe von Wirkstoffen über den Anwendungszeitraum eine gute Beständigkeit aufweist und somit über diesen Zeitraum ihre Wirkung entfalten kann.

Es zeigte sich darüber hinaus überraschenderweise, dass die erfindungsgemäßen Polymerzusammensetzungen, insbesondere, wenn sie Cellulose enthalten beziehungsweise Cellulose-basiert sind, zu Fasern führen, die trotz des Zusatzes mindestens einem der erfindungsgemäßen Additive dieselben hervorragenden Eigenschaften zeigen, wie reine Fasern, insbesondere Cellulosefasern, beispielsweise bezüglich ihrer Feinheit, Reißkraft, Reißkraftvariation, Dehnung, Nassdehnung, feinheitsbezogenen Reißkraft, feinheitsbezogenen Nassreißkraft, feinheitsbezogenen Schlingenreißkraft, Nassscheuerungsvariation und Nassmodulen. Dennoch zeigten diese Fasern gleichzeitig die durch das mindestens eine Additiv verliehenen positiven Eigenschaften.

Bevorzugt werden die Fasern aus einer Spinnlösung hergestellt. Es zeigte sich, dass trotz der Zugabe mindestens eines erfindungsgemäßen Additivs überraschenderweise keine Zersetzung einer Spinnlösung, insbesondere, wenn sie Cellulose enthält, und ganz besonders wenn die Spinnlösung aus Cellulose, N-Methylmorpholin-N-Oxid (NMMNO) und Wasser als Hauptkomponenten gebildet wird.

Geeignete Herstellverfahren, insbesondere für Polymerzusammensetzungen, die Cellulose oder modifizierte Cellulose enthalten, und insbesondere in Form von Formkörpern sind aus der DE 10 2007 054 702 A1 bekannt.

Die Polymerzusammensetzung kann Polymere enthalten, wie sie typischer weise für die Herstellung von Fasern, Filamenten, Garnen, Vliesstoffen, Kleidungsstücken und medizinischen Hilfsmitteln wie Bandagen und Kompressionsstrümpfen verwendet werden.

In einer bevorzugten Ausführungsform ist das Polymer ausgewählt aus der Gruppe bestehend aus Cellulose, modifizierte Cellulose, Polyamid, Polyacryl, Polyester, Polyurethan, Polyethylen, Polypropylen, Modifikationen davon und Mischungen davon. In einer bevorzugten Ausführungsform ist das Polymer ausgewählt aus der Gruppe bestehend aus Cellulose, modifizierte Cellulose, Polyamid, Polyacryl, Polyester, Modifikationen davon und Mischungen davon.

In einer bevorzugten Ausführungsform ist das Polymer biologisch abbaubar. Das biologisch abbaubare Polymer ist bevorzugt ausgewählt aus der Gruppe bestehend aus Cellulose, modifizierter Cellulose, Latex, Eiweiß pflanzlicher sowie tierischer Herkunft sowie Gemischen davon. Auch können Polykondensations- und Polymerisations-Polymere, Polyurethane, Polyester, Polyacryle und Gemische von diesen Materialien verwendet werden.

In einer alternativen Ausführungsform kann jedoch auch vorgesehen sein, dass die erfindungsgemäße Polymerzusammensetzung auch nicht-biologisch abbaubare Polymere enthält. Beispiele solcher Polymere sind Polyamide, aromatische Polyamide, insbesondere Aramide, Polyacrylnitril, Polyester oder Polyvinylalkohole.

In einer bevorzugten Ausführungsform ist das Polymer Cellulose, modifizierte Cellulose, oder Modifikationen davon und Mischungen davon. In einer bevorzugten Ausführungsform ist das Polymer Cellulose oder modifizierte Cellulose.

Bei Cellulose handelt es sich um ein ein hydrophiles Netzwerk ausbildendes Polymer, dessen supramolekulare Strukturen sowohl in Lösung als auch im Festkörper über ebenfalls hydrophile Wasserstoff-Brückenbindungen stabilisiert sind. Dagegen sind die in der vorliegenden Erfindung verwendeten Wirkstoffe ausgewählt aus der Gruppe bestehend aus eine innere Okklusion erzeugende Wirkstoffe, eine äußere Okklusion erzeugende Wirkstoffe und Feuchtigkeitsspendende Wirkstoffe eher unpolare, lipophile organische Verbindungen. Es zeigte sich nun im Rahmen der vorliegenden Erfindung überraschenderweise, dass sich solche unpolaren, lipophilen Wirkstoffe in die cellulosischen Filamente und Fasern einbringen lassen. Ebenso überraschend ist es, dass die in dem hydrophilen Cellulosenetzwerk inkorporierten und polaren lipophilen organischen Wirkstoffe sich derart homogen und mikropartikulär verteilen lassen, dass eine feindosierbare und gleichmäßige Speicherung sowie kontrollierte Freisetzung dieser unpolaren lipophilen organischen Wirkstoffe aus den Fasern ermöglicht wird.

Bevorzugt wird die erfindungsgemäße Polymerzusammensetzung aus einer Spinnlösung hergestellt. Bevorzugt liegen die Wirkstoffe in der Polymerzusammensetzung als Mikroeinschlüsse vor.

Unter modifizierter Cellulose wird insbesondere Carboxyethyl-Cellulose, Methyl-Cellulose, Nitrat-Cellulose, Kupfer-Cellulose, Viskose, also Cellulose-Xantoghenat, Cellulosecarbamat und Celluloseacetat verstanden.

In einer alternativen Ausführungsform ist das Polymer ein Polyamid, ein Polyacryl, ein Polyester oder Modifikationen davon und Mischungen davon. In einer bevorzugten Ausführungsform ist das Polymer ein Polyamid, oder Modifikationen davon. In einer bevorzugten Ausführungsform ist das Polymer ein Polyacryl oder Modifikationen davon. In einer bevorzugten Ausführungsform ist das Polymer ein Polyester oder Modifikationen davon.

Beispiele für Polykondensations- und Polymerisations-Polymere sind Polyamide, die zum Beispiel mit Methyl-, Hydroxi-, oder Benzylgruppen substituiert sind. Beispiele für Polyurethane sind solche, die auf der Basis von Polyether-Polyolen aufgebaut sind.

Erfindungsgemäß enthält die Polymerzusammensetzung mindestens einen Wirkstoff, wobei es sich bei dem Wirkstoff um einen eine Okklusion erzeugenden Wirkstoff, insbesondere einen eine innere Okklusion erzeugenden Wirkstoff oder einen eine äußere Okklusion erzeugenden Wirkstoff, um einen feuchtigkeitsspendenden Wirkstoff, oder um einen Schmerz oder Juckreiz reduzierenden Wirkstoff oder Mischungen hiervon handeln kann.

Bevorzugt ist der mindestens eine Wirkstoff in die Polymerzusammensetzung inkorporiert und insbesondere nicht durch Tränkung oder Beschichtung auf die Trägermatrix aufgetragen.

Bevorzugt enthält die Polymerzusammensetzung mindestens einen eine Okklusion erzeugenden Wirkstoff, insbesondere einen eine innere Okklusion erzeugenden Wirkstoff oder einen eine äußere Okklusion erzeugenden Wirkstoff.

Im Zusammenhang mit der vorliegenden Erfindung wird unter Okklusion die Verhinderung oder Reduzierung von Wasserverlust durch die Haut an die Umgebung, wie beispielsweise einer textilen Auflage, verstanden.

Neben weiteren wichtigen Funktionen schützt die menschliche Haut vor Angriffen aus der Umwelt. Zur Aufrechterhaltung ihrer Schutzfunktion ist es von wesentlicher Bedeutung, den Verlust von Wasser aus den tieferen Hautschichten zu vermeiden.

Der Aufbau der menschlichen Haut gliedert sich in drei Bestandteile, nämlich die Oberhaut (Epidermis), die Lederhaut (Dermis) und die Unterhaut (Subcutis). Eine besondere Rolle für den Wassergehalt der Haut spielen die äußeren Schichten des Stratum Corneum der Epidermis mit ihren Keratinfilamenten. So kann das Keratinfasergerüst beträchtliche Wassermengen halten. Ziel- und Angriffspunkt der äußeren okklusiven Wirkung eines entsprechenden Stoffes ist die Epidermis. Durch Aufbringen eines solchen Stoffes auf die Hautoberfläche entfaltet sich die Hauptwirkung, nämlich die Unterdrückung eines Entweichens von Wasser (trans-epidermaler Wasserverlust) aus dem Stratum Corneum. Durch diese Stoffe wird ein Film gebildet, der das Entweichen von Feuchtigkeit verhindert. Bei der äußeren Okklusion kann somit Wasser aus den tieferen Schichten nachrücken und das Keratinfasergerüst der äußeren Hautschicht mit Feuchtigkeit auffüllen. Dadurch schützt der Stratum Corneum vor Austrockung. Elastizität und Geschmeidigkeit der Haut werden erhalten und die Haut erhält ihr physiologisches Gleichgewicht zum Schutz beispielsweise vor Pilzbefall. Diese okklusive Wirkweise eines Wirkstoffes wird auch als äußere Okklusion bezeichnet

Die innere Okklusion bezeichnet eine Änderung der Hautlipidekonfiguration im Stratum Corneum. In der hexagonalen Konfiguration können die Kohlenwasserstoffketten der Lipide frei um ihre Achse rotieren. Diese Phase der "lockeren Packung" schafft eine durchlässigere, also permeable Struktur und erleichtert eine unerwünschte Wasserabgabe unter Stress im Stratum Corneum mit entsprechenden nachteiligen Folgen, wie beispielsweise Hauttrockenheit. Durch die Einwirkung bestimmter Wirkstoffe wird bei der inneren Okklusion eine dichtere ortho-rhombische Packung der Lipide erzeugt. Diese Anordnung führt zu einer festeren Struktur. Solch eine molekulare Aktion bewirkt durch die "ortho-rhombische Packung" der Lipide eine wesentlich geringere Durchlässigkeit der Hautbarriere und reduziert somit signifikant den Wasserverlust aus dem Stratum Corneum.

In einer Ausführungsform erzeugt der mindestens eine Wirkstoff eine innere Okklusion.

Bevorzugt ist der mindestens eine eine innere Okklusion erzeugende Wirkstoff ein Fettsäurederivat. Bevorzugt ist das Fettsäurederivat ausgewählt aus der Gruppe bestehend aus Isostearyl Isostearat, Isopropyl Palmitat und Mischungen davon. Bevorzugt ist das Fettsäurederivat Isostearyl Isostearat. Alternativ ist das Fettsäurederivat Isopropyl Palmitat.

In einer alternativen Ausführungsform erzeugt der mindestens eine Wirkstoff eine äußere Okklusion.

Bevorzugt ist der mindestens eine eine äußere Okklusion erzeugende Wirkstoff ausgewählt aus der Gruppe bestehend aus höhermolekulare Alkansäuren, höhermolekulare Alkensäuren, höhermolekulare Carbonate und Mischungen davon. Bevorzugt ist der mindestens eine eine äußere Okklusion erzeugende Wirkstoff ausgewählt aus der Gruppe bestehend aus Cetearyl Isononanoat, Dicaprylyl Carbonat und Mischungen davon. Bevorzugt ist der Wirkstoff Cetearyl Isononanoat. Bevorzugt ist der Wirkstoff Dicaprylyl Carbonat.

In einer alternativen Ausführungsform ist der mindestens eine Wirkstoff ein feuchtigkeitsspendender Wirkstoff.

Bevorzugt ist der mindestens eine feuchtigkeitsspendende Wirkstoff ausgewählt aus der Gruppe bestehend aus Glycerin, Polyolen, insbesondere Panthenol, und anderen feuchtigkeitsspendenden Stoffen wie Harnstoff. Bevorzugt ist der mindestens eine feuchtigkeitsspendende Wirkstoff Glycerin (Propan-1,2,3-triol).

Bevorzugt ist der mindestens eine Schmerz oder Juckreiz reduzierenden Wirkstoff eine Fettsäureamidverbindung, eine Modifikationen davon oder ein Derivat davon oder ein Flavonoid, eine Modifikationen davon oder ein Derivat davon.

Bevorzugt ist der mindestens eine Schmerz oder Juckreiz reduzierenden Wirkstoff ausgewählt aus der Gruppe bestehend aus Palmitoylethanolamin (PEA), Stearoylethanolamid (SEA), Oxerutin, Troxerutin und Mischungen davon.

Bevorzugt ist der mindestens eine Schmerz oder Juckreiz reduzierenden Wirkstoff ein N-Acylethanolamin (NAE).

Bevorzugt ist der mindestens eine Schmerz oder Juckreiz reduzierenden Wirkstoff Palmitoylethanolamin (PEA) oder Stearoylethanolamid (SEA).

Die natürlich vorkommenden Fettamide PEA und seine Homologe wie das eng verwandte SEA weisen sehr gute anti-inflammatorische Eigenschaften auf. So werden sie als Lipid-Komponente in kosmetischen Formulierungen wie beispielsweise Haarwasch-Produkten gegen austrocknende Kopfhaut verwendet. Ein Einsatz dieser Stoffe erfolgt aber auch beispielsweise gegen Grippeviren, Erkältungskrankheiten, Erkrankungen der Atemwege und Rheumatisches Fieber. Auch werden die Stoffe als orale Gabe mit schmerzlindernder Eigenschaft verabreicht. Allgemein zeigen diese Stoffe eine Juckreiz mildernde, schmerzlindernde und entzündungshemmende Wirkung. Die menschliche Oberhaut kann sowohl durch schädliche Umwelteinflüsse als auch durch permanente Irritationen ausgelöst beispielsweise über textile Oberflächen angegriffen werden. Als Folge daraus entsteht die subjektive Empfindung von Juckreiz und Schmerz. Der physiologische Wirkmechanismus kann wie folgt erklärt werden: Schmerz- und Entzündungsreize (beispielsweise ausgelöst durch eine textile Oberfläche) treffen auf Rezeptoren der Schmerzweiterleitung und die Aktivierung der Entzündungs-Rezeptoren der Mastzellen führt zur Ausschüttung von stark entzündlich wirkenden Histaminen. Ergebnis ist zum einen die Auslösung der Schmerzempfindung durch Weiterleitung in den sensorischen Nervenfasern der Haut bis in die maßgeblichen Hirngebiete. Zum Zweiten führt die Ausschüttung der Entzündungsstoffe in der Epidermis zu einem Juckreiz, Rötungen und zu Trockenheit der Haut. Linderung auf molekularer Ebene schafft der Angriff von PEA und seinen Homologen aufgrund zweier Mechanismen: Zum einen handelt es sich bei PEA und seinen Homologen um einen Mastzell-Modulator. Die Histamin-Ausschüttung der Mastzellen wird durch erhöhte PEA-Konzentrationen am Ort der Entzündung gehemmt. Zum anderen haben PEA und seine Homologe eine hohe Affinität zu den für Schmerz und Entzündung verantwortlichen Rezeptoren. Dies führt zu einer Unterbindung der entsprechenden Reizleitung. So zeigen PEA und seine Homologe bei Einwirkung auf betroffene Hautgebiete eine entzündungshemmende, beruhigende Wirkung für trockene juckende und gereizte Haut.

Bevorzugt ist der mindestens eine Schmerz oder Juckreiz reduzierenden Wirkstoff ein Flavonoid, eine Modifikationen davon oder ein Derivat davon. Bevorzugt ist der mindestens eine Schmerz oder Juckreiz reduzierenden Wirkstoff ein Rutin, eine Modifikationen davon oder ein Derivat davon.

Bevorzugt ist der mindestens eine Schmerz oder Juckreiz reduzierenden Wirkstoff Rutin, Oxerutin oder Troxerutin, eine Modifikationen davon oder ein Derivat davon. Bevorzugt ist der mindestens eine Schmerz oder Juckreiz reduzierenden Wirkstoff Troxerutin, eine Modifikationen davon oder ein Derivat davon. Bevorzugt ist der mindestens eine Schmerz oder Juckreiz reduzierenden Wirkstoff Troxerutin.

Troxerutin ist ein pflanzlicher Wirkstoff aus der Gruppe der Flavonoide und wird bei Krankheiten eingesetzt, die mit einer erhöhten Durchlässigkeit der Blutgefäße einhergehen. Zu nennen sind hier Venenerkrankungen, die mit Wassereinlagerungen im Gewebe, also Ödemen einhergehen. Dazu gehören beispielsweise oberflächliche Venenentzündungen oder Krampfadern sowie die Venenschwächen während der Schwangerschaft. Auch bei offenen Beinen, Chronisch-venöser Insuffizienz und bei schweren, müden Beinen oder Beinkrämpfen, die auf eine Verstopfung der Beinvenen zurückzuführen sind, werden Rutine wie beispielsweise Troxerutin angewandt. Bei Stauungen in den Venen entstehen neben einer Schädigung der Gefäßinnenwände Blutklümpchen, Verstopfungen und Entzündungen. Gefäßinnenwandsschädigungen werden durch Stoffe verursacht, die die weißen Blutkörperchen im Rahmen der Entzündung freisetzen. Troxerutin verhindert die Abgabe dieser Stoffe und stabilisiert so die durchlässigen Gefäßwände der Blutkapillaren. Diese werden derart abgedichtet, dass keine Flüssigkeit mehr austreten kann. Außerdem verbessert Troxerutin die Fließeigenschaften des Blutes, indem es die roten Blutkörperchen verformbarer macht und eine leicht gerinnungshemmende Wirkung hat. Die Summe dieser Effekte trägt dazu bei, dass bei Blutstauungen in den Venen weniger Wasser in das umliegende Gewebe austritt und Ödeme bildet. Symptome der Venenerkrankungen wie Schmerzen und Schweregefühl in den Beinen werden auf diese Art durch die Anwendung von Troxerutin gelindert.

In einer alternativen Ausführungsform enthält die Polymerzusammensetzung mindestens einen eine Okklusion erzeugender Wirkstoff, insbesondere einen eine innere Okklusion erzeugenden Wirkstoff oder einen eine äußere Okklusion erzeugenden Wirkstoff, und mindestens einen Schmerz oder Juckreiz reduzierenden Wirkstoff.

In einer alternativen Ausführungsform enthält die Polymerzusammensetzung mindestens einen feuchtigkeitsspenden Wirkstoff und mindestens einen Schmerz oder Juckreiz reduzierenden Wirkstoff.

In einer alternativen Ausführungsform enthält die Polymerzusammensetzung mindestens einen eine Okklusion erzeugender Wirkstoff, insbesondere einen eine innere Okklusion erzeugenden Wirkstoff oder einen eine äußere Okklusion erzeugenden Wirkstoff, und mindestens einen feuchtigkeitsspendenden Wirkstoff.

In einer alternativen Ausführungsform enthält die Polymerzusammensetzung mindestens einen eine Okklusion erzeugender Wirkstoff, insbesondere einen eine innere Okklusion erzeugenden Wirkstoff oder einen eine äußere Okklusion erzeugenden Wirkstoff, mindestens einen Schmerz oder Juckreiz reduzierenden Wirkstoff und mindestens einen feuchtigkeitsspendenden Wirkstoff.

Die vorliegende Erfindung betrifft auch einen Formkörper oder eine Vielzahl von Formkörpern umfassend eine erfindungsgemäße Polymerzusammensetzung.

Ein Vorteil der erfindungsgemäßen Formkörper, in denen die erfindungsgemäßen Additive enthalten sind, ist der gleichmäßige Einbau der Wirkstoffe in dem Formkörper, insbesondere in die Fasermatrix, bei unterschiedlich herstellbaren Faserquerschnitten. Dabei ist auch die Verarbeitung als Monofilament- oder Endlosfilamentgarn möglich, wodurch sich eine Einsatzmöglichkeit bei technischen Artikeln ergibt.

Die erfindungsgemäßen Formkörper können für verschiedenste Zwecke verwandt werden.

Bevorzugt ist der Formkörper eine Faser. Bevorzugt ist die Faser eine Stapelfaser, ein Monofilament, ein multifiles Filament oder ein multifiles Endlosfilament.

Insbesondere wenn die Faser eine cellulosische Faser ist, kann sie beispielsweise wie in den Figuren 1 und 2 der DE 10 2007 054 702 A1 gezeigt aufgebaut sein. Der mindestens eine Wirkstoff kann in Form von dispergierten Einschlüssen in einer Matrix, insbesondere Cellulosematrix vorliegen.

Bevorzugt ist der mindestens eine Wirkstoff mit zumindest einem hydrophoben Verdickungsmittel stabilisiert.

Insbesondere, wenn aus den erfindungsgemäßen Fasern eine textile Fläche hergestellt wird, kann dieses nicht nur aus der erfindungsgemäßen Faser bestehen, sondern auch zusätzliche Komponenten enthalten, beispielsweise Baumwolle, Lyocell, Rayon, Carbacell, Polyester, Polyamid, Celluloseacetat, Acrylat, Polypropylen oder Gemische davon.

Die vorliegende Erfindung betrifft auch die Verwendung eines erfindungsgemäßen Formkörpers zur Herstellung von Garnen.

Die vorliegende Erfindung betrifft auch ein Garn, enthaltend einen erfindungsgemäßen Formkörper. Die vorliegende Erfindung betrifft auch ein Garn, enthaltend die erfindungsgemäßen Formkörper als eine Grundkomponente. Die vorliegende Erfindung betrifft auch ein Garn, bestehend aus erfindungsgemäßen Formkörpern.

Die vorliegende Erfindung betrifft auch einen Vliesstoff, enthaltend einen erfindungsgemäßen Formkörper. Die vorliegende Erfindung betrifft auch einen Vliesstoff, enthaltend ein erfindungsgemäßes Garn.

Die vorliegende Erfindung betrifft auch ein Kleidungstück oder ein medizinisches Hilfsmittel, enthaltend einen erfindungsgemäßen Formkörper oder ein erfindungsgemäßes Garn. Bevorzugt handelt es sich um Kleidungsstücke oder medizinische Hilfsmittel, die beim Tragen zumindest teilweise direkt an der Haut anliegen.

Die vorliegende Erfindung betrifft auch ein Kleidungstück, enthaltend einen erfindungsgemäßen Formkörper oder ein erfindungsgemäßes Garn. Bevorzugt ist das Kleidungsstück Unterwäsche, insbesondere eine Unterhose, ein Unterhemd oder ein Strumpf. Alternativ kann es sich bei dem Kleidungsstück auch um einen Handschuh handeln.

Die vorliegende Erfindung betrifft auch ein medizinisches Hilfsmittel, enthaltend einen erfindungsgemäßen Formkörper oder ein erfindungsgemäßes Garn. Bevorzugt handelt es sich bei dem medizinischen Hilfsmittel um einen Kompressionsstrumpf oder um eine Bandage.

Die vorliegende Erfindung betrifft auch eine Sportbandage und andere Sporthilfsmittel, enthaltend einen erfindungsgemäßen Formkörper oder ein erfindungsgemäßes Garn.

Die vorliegende Erfindung betrifft auch die Verwendung einer erfindungsgemäßen Polymerzusammensetzung oder eines erfindungsgemäßen Formkörpers in einem Kleidungstück, einem Sporthilfsmittel oder in einem medizinischen Hilfsmittel zur Pflege der Haut.

Die vorliegende Erfindung betrifft auch die Verwendung einer erfindungsgemäßen Polymerzusammensetzung oder eines erfindungsgemäßen Formkörpers in einem Kleidungstück, einem Sporthilfsmittel oder in einem medizinischen Hilfsmittel zur Verhinderung des Austrocknens der Haut.

Die vorliegende Erfindung betrifft auch die Verwendung einer erfindungsgemäßen Polymerzusammensetzung oder eines erfindungsgemäßen Formkörpers in einem Kleidungstück, einem Sporthilfsmittel oder in einem medizinischen Hilfsmittel zur Reduzierung von Schmerz und/oder Juckreiz, insbesondere bei geschädigter Haut und bei Hautkrankheiten, wie beispielsweise bei Neurodermitis. Bevorzugt wird dabei gleichzeitig ein Austrocknen der Haut reduziert oder verhindert.

Die vorliegende Erfindung betrifft auch folgende Aspekte:
Aspekt 1. Polymerzusammensetzung, enthaltend ein Polymer und mindestens einen Wirkstoff ausgewählt aus der Gruppe bestehend aus einem eine Okklusion erzeugenden Wirkstoff, insbesondere einem eine innere Okklusion erzeugenden Wirkstoff oder einem eine äußere Okklusion erzeugenden Wirkstoff, einem feuchtigkeitsspendenden Wirkstoff, einem Schmerz oder Juckreiz reduzierenden Wirkstoff und Mischungen davon.
Aspekt 2. Polymerzusammensetzung nach Aspekt 1, wobei das Polymer ausgewählt ist aus der Gruppe bestehend aus Cellulose, modifizierte Cellulose, Polyamid, Polyacryl, Polyester, Polyurethan, Polyethylen, Polypropylen, Modifikationen davon, Derivaten davon und Mischungen davon.
Aspekt 3. Polymerzusammensetzung nach einem der vorhergehenden Aspekte, wobei der mindestens eine Wirkstoff ein unpolarer Wirkstoff ist.
Aspekt 4. Polymerzusammensetzung nach einem der vorhergehenden Aspekte, wobei der mindestens eine eine innere Okklusion erzeugende Wirkstoff ein Fettsäurederivat ist, insbesondere ausgewählt ist aus der Gruppe bestehend aus Isostearyl Isostearat, Isopropyl Palmitat und Mischungen davon.
Aspekt 5. Polymerzusammensetzung nach einem der vorhergehenden Aspekte, wobei der mindestens eine eine äußere Okklusion erzeugende Wirkstoff ausgewählt ist aus der Gruppe bestehend aus höhermolekulare Alkansäuren, höhermolekulare Alkensäuren, höhermolekulare Carbonate und Mischungen davon, insbesondere Cetearyl Isononanoat, Dicaprylyl Carbonat und Mischungen davon.
Aspekt 6. Polymerzusammensetzung nach einem der vorhergehenden Aspekte, wobei der mindestens eine feuchtigkeitsspendende Wirkstoff ausgewählt ist aus der Gruppe bestehend aus Glycerin, Polyolen, insbesondere Panthenol, und Harnstoff.
Aspekt 7. Polymerzusammensetzung nach einem der vorhergehenden Aspekte, wobei der mindestens eine feuchtigkeitsspendende Wirkstoff Glycerin ist.
Aspekt 8. Polymerzusammensetzung nach einem der vorhergehenden Aspekte, wobei der mindestens eine Schmerz oder Juckreiz reduzierenden Wirkstoff eine Fettsäureamidverbindung, eine Modifikationen davon oder ein Derivat davon oder ein Flavonoid, eine Modifikationen davon oder ein Derivat davon ist.
Aspekt 9. Polymerzusammensetzung nach einem der vorhergehenden Aspekte, wobei der mindestens eine Schmerz oder Juckreiz reduzierenden Wirkstoff ausgewählt ist aus der Gruppe bestehend aus Palmitoylethanolamin (PEA), Stearoylethanolamid (SEA), Oxerutin, Troxerutin und Mischungen davon.
Aspekt 10. Formkörper, umfassend eine Polymerzusammensetzung nach einem der vorhergehenden Aspekte.
Aspekt 11. Formkörper nach Aspekt 10, wobei der Formkörper eine Faser ist.
Aspekt 12. Formkörper nach Aspekt 11, wobei die Faser eine Stapelfaser, ein Monofilament, ein multifiles Filament oder ein multifiles Endlosfilament ist.
Aspekt 13. Verwendung des Formkörpers nach einem der Aspekte 10 bis 12 zur Herstellung von Garnen oder textilen Flächen, insbesondere Filze oder Vliesen.
Aspekt 14. Garn, enthaltend einen Formkörper nach einem der Aspekte 10 bis 12.
Aspekt 15. Vliesstoff, enthaltend einen Formkörper nach einem der Aspekte 10 bis 12 oder ein Garn nach Aspekt 14.
Aspekt 16. Kleidungstück oder medizinisches Hilfsmittel, enthaltend einen Formkörper nach einem der Aspekte 10 bis 12 oder ein Garn nach Aspekt 14.
Aspekt 17. Verwendung einer Polymerzusammensetzung nach einem der Aspekte 1 bis 9 oder eines Formkörpers nach einem der Aspekte 10 bis 12 oder eines Garns nach Aspekt 14 oder einem Vliesstoff nach Aspekt 15 in einem Kleidungstück, in einem Sporthilfsmittel oder in einem medizinischen Hilfsmittel zur Pflege der Haut.

## Patentansprüche

1. Garn, enthaltend einen Formkörper umfassend eine Polymerzusammensetzung, enthaltend ein Polymer und einen Schmerz oder Juckreiz reduzierenden Wirkstoff ausgewählt ist aus der Gruppe bestehend aus Palmitoylethanolamin (PEA), Stearoylethanolamid (SEA) und Mischungen davon, **dadurch gekennzeichnet, dass** das Polymer ausgewählt ist aus der Gruppe bestehend aus Polyamid, Polyacryl, Polyester, Polyurethan, Polyethylen, Polypropylen, Modifikationen davon, Derivaten davon und Mischungen davon.

2. Garn nach Anspruch 1, wobei das Polymer Polyamid oder Modifikationen davon ist.

3. Garn nach einem der vorhergehenden Ansprüche, wobei die Polymerzusammensetzung zusätzlich mindestens einen eine innere Okklusion erzeugenden Wirkstoff enthält, ausgewählt aus der Gruppe bestehend aus Isostearyl Isostearat, Isopropyl Palmitat und Mischungen davon.

4. Garn nach einem der vorhergehenden Ansprüche, wobei die Polymerzusammensetzung zusätzlich mindestens eine eine äußere Okklusion erzeugende Wirkstoff enthält, ausgewählt aus der Gruppe bestehend aus höhermolekulare Alkansäuren, höhermolekulare Alkensäuren, höhermolekulare Carbonate und Mischungen davon, insbesondere Cetearyl Isononanoat, Dicaprylyl Carbonat und Mischungen davon.

5. Garn nach einem der vorhergehenden Ansprüche, wobei der mindestens eine Schmerz oder Juckreiz reduzierenden Wirkstoff Palmitoylethanolamin (PEA) ist.

6. Garn nach einem der vorhergehenden Ansprüche, wobei der Formkörper eine Faser ist.

7. Garn nach Anspruch 6, wobei die Faser eine Stapelfaser, ein Monofilament, ein multifiles Filament oder ein multifiles Endlosfilament ist.

8. Vliesstoff, enthaltend ein Garn nach einem der vorhergehenden Ansprüche.

9. Kleidungstück oder medizinisches Hilfsmittel, enthaltend ein Garn nach einem der Ansprüche 1 bis 7 oder ein Vliesstoff nach Anspruch 8.

10. Medizinisches Hilfsmittel nach Anspruch 9, wobei es sich bei dem medizinischen Hilfsmittel um einen Kompressionsstrumpf oder um eine Bandage handelt.

11. Verwendung eines Garns nach einem der Ansprüche 1 bis 7 oder einem Vliesstoff nach Anspruch 8 in einem Kleidungstück, in einem Sporthilfsmittel oder in einem medizinischen Hilfsmittel zur Pflege der Haut.
